# EUROPEAN PATENT APPLICATION

(11) **EP 4 501 350 A1**
(43) Date of publication of application: **05.02.2025**
(21) Application number: 23780505.6
(22) Date of filing: 28.03.2023
(51) Int. Cl.: A61K 39/00, A61K 39/395, C07K 16/18, C12N 5/10, A61K 47/64, C12N 1/15, C12N 1/19, C12N 1/21, C12N 15/13, C12N 15/63, A61P 25/04

(54) **ANTI-NETRIN-4 ANTIBODY AND USE THEREOF**

(30) Priority: 30.03.2022 JP 2022056428
(71) Applicant: OSAKA UNIVERSITY, Suita-shi Osaka 565-0871 (JP)
(72) Inventor: YAMASHITA, Toshihide, Suita-shi, Osaka 565-0871 (JP)
(74) Representative: Kraus & Lederer PartGmbB
(86) International application number: PCT/JP2023/012470
(87) International publication number: WO 2023/190496

(57) **Abstract**

The present invention provides an anti-Netrin-4 antibody that recognizes, as an epitope, an amino acid sequence of residues 192 to 202 (SEQ ID NO: 2) in an amino acid sequence of human Netrin-4 (SEQ ID NO: 1); and a pharmaceutical composition for preventing or treating pain, comprising the antibody or a functional fragment thereof as an active ingredient.

## Description

### TECHNICAL FIELD

The present invention relates to an anti-human Netrin-4 antibody and use thereof.

### BACKGROUND ART

Pain has a great impact on the physiological functions of the body and the mental state of patients and reduces their QOL (Quality Of Life) due to its severity. The number of patients with chronic pain in the world is reported to exceed 20 million, and the overall market size of medicines for pain treatment in Japan, the U.S. and Europe is said to be about 2 trillion yen. In addition, the number of patients with diseases that may cause pain, such as cancer, stroke, diabetes mellitus and AIDS, has been increasing, and under such circumstances, the establishment of an appropriate treatment strategy for pain is a very important medical issue. Particularly, neuropathic pain is less sensitive to nonsteroidal anti-inflammatory drugs and narcotic analgesics, and medical needs for the development of more effective medicines for neuropathic pain are expected to increase. However, the pathogenesis of neuropathic pain is diverse and the underlying molecular mechanism is very complicated. Due to these reasons, a medicine for radical treatment of neuropathic pain is yet to be developed (non patent literature 1). Clarifying the molecular mechanism of the onset and persistence of neuropathic pain to promote the development of a breakthrough medicine for neuropathic pain is one of the biggest medical issues in the 21st century.

One of the causes of neuropathic pain is believed to be plastic changes in neural circuits in the dorsal horn, which is present in the dorsum of the spinal cord (non patent literature 2). Sensory inputs from the periphery undergo various processing, such as amplification, attenuation and integration, in the dorsal horn of the spinal cord and are transmitted to the brain. Peripheral nerve injuries reportedly induce plastic changes in the neural network in the dorsal horn of the spinal cord, including, for example, abnormal sprouting of axon collaterals and enhanced synaptic transmission, leading to the onset of pain (non patent literature 3). Clarifying the molecular mechanism that regulates the plasticity of neural network in the dorsal horn is expected to promote the development of a novel pain therapy.

Netrin-4 is a member of the secreted protein Netrin family. Netrin-4 has a structure very similar to that of the β-chain of extracellular matrix laminin, and is known to have various roles, including those associated with neurite formation, cell migration, cell survival, angiogenesis, and cancer cell growth (non patent literature 4). The inventors developed a pain model using Netrin-4 knockout animals (rats), and examined responses to mechanical stimulation by the von Frey filament test to clarify whether Netrin-4 is involved in the onset of pain. Hyperalgesia conditions were not observed in the neuropathic pain model or the chronic inflammation pain model as compared with wild-type animals. These experiments revealed, for the first time, possible involvement of Netrin-4 in the onset of various types of pain (non patent literature 5 and patent literature 1).

### CITATION LIST

### PATENT LITERATURE

Patent literature 1: WO2015/025770

### NON PATENT LITERATURE

Non patent literature 1: Dworkin RH, O'Connor AB, Backonja M, Farrar JT, Finnerup NB, Jensen TS, Kalso EA, Loeser JD, Miaskowski C, Nurmikko TJ, Portenoy RK, Rice AS, Stacey BR, Treede RD, Turk DC, Wallace MS: Pharmacologic management of neuropathic pain: evidence-based recommendations. Pain. 2007 Dec 5;132(3):237-251.
Non patent literature 2: Woolf CJ, Salter MW: Neuronal plasticity: increasing the gain in pain. Science. 2000 Jun 9;288(5472):1765-1769.
Non patent literature 3: Markman JD, Dworkin RH: Ion channel targets and treatment efficacy in neuropathic pain. Journal of Pain 2006;7(1):S38-S47.
Non patent literature 4: Lai Wing Sun K, Correia JP, Kennedy TE: Netrins: versatile extracellular cues with diverse functions. Development. 2011 Jun;138(11):2153-2169.
Non patent literature 5: Hayano, Y., Takasu, K., Koyama, Y., Ogawa, K., Minami, K., Asaki, T., Kitada, K., Kuwabara, S. and Yamashita, T.: Dorsal horn interneuron-derived Netrin-4 contributes to spinal sensitization in chronic pain via Unc5B 2016 Dec 12;213(13):2949-2966.

### SUMMARY OF INVENTION

### TECHNICAL PROBLEM

An object of the present invention is to provide an antibody that binds to Netrin-4 and is useful for prevention or treatment of pain, and a pharmaceutical composition for preventing or treating pain, comprising the antibody as an active ingredient.

### SOLUTION TO PROBLEM

The present invention was made to solve the above problems and includes the following.
[1] An anti-Netrin-4 antibody that recognizes, as an epitope, an amino acid sequence of residues 192 to 202 (SEQ ID NO: 2) in an amino acid sequence of human Netrin-4 (SEQ ID NO: 1).
[2] The anti-Netrin-4 antibody according to the above [1], wherein the antibody comprises the following (a) and (b):
   (a) a heavy chain variable region comprising a heavy chain CDR1 of the amino acid sequence of SEQ ID NO: 7, a heavy chain CDR2 of the amino acid sequence of SEQ ID NO: 8, and a heavy chain CDR3 of the amino acid sequence of SEQ ID NO: 9; and
   (b) a light chain variable region comprising a light chain CDR1 of the amino acid sequence of SEQ ID NO: 10, a light chain CDR2 of the amino acid sequence of SEQ ID NO: 11, and a light chain CDR3 of the amino acid sequence of SEQ ID NO: 12.
[3] The antibody according to the above [1] or [2], wherein the antibody is a human chimeric antibody or a humanized antibody.
[4] The antibody according to the above [2], wherein the antibody is produced by a hybridoma cell line, Human Netrin-4(192-202)-6A1 (Accession No. NITE BP-03603).
[5] A hybridoma cell line, Human Netrin-4(192-202)-6A1 (Accession No. NITE BP-03603).
[6] A functional fragment of the antibody according to the above [1] to [4].
[7] A polynucleotide encoding the heavy chain variable region of the antibody according to the above [1] to [4].
[8] A polynucleotide encoding the light chain variable region of the antibody according to the above [1] to [4].
[9] An expression vector comprising the polynucleotide according to the above [7] and/or the above [8].
[10] A transformed cell comprising the expression vector according to the above [9].
[11] A pharmaceutical composition for preventing or treating pain, comprising the antibody according to the above [1] to [4] or the functional fragment according to the above [6] as an active ingredient.
[12] The pharmaceutical composition according to the above [11], wherein the composition is for administration to dorsal root ganglion.
[13] A vaccine composition for preventing or treating pain, comprising a peptide comprising an amino acid sequence of residues 192 to 202 (SEQ ID NO: 2) in an amino acid sequence of human Netrin-4 (SEQ ID NO: 1), and a carrier protein.

### ADVANTAGEOUS EFFECTS OF INVENTION

The present invention provides an antibody that binds to Netrin-4 and is useful for prevention or treatment of pain. The present invention also provides a pharmaceutical composition for preventing or treating pain, comprising the antibody as an active ingredient.

### BRIEF DESCRIPTION OF DRAWINGS

Fig. 1 shows the evaluation results of changes in nociceptive responses after intrathecal administration of an anti-Netrin-4 polyclonal antibody to neuropathic pain model rats.
Fig. 2 shows the evaluation results of changes in nociceptive responses after intrathecal administration of an anti-Netrin-4 monoclonal antibody to neuropathic pain model rats.
Fig. 3 shows the evaluation results of changes in nociceptive responses after administration of an anti-Netrin-4 monoclonal antibody to the dorsal root ganglion of neuropathic pain model rats.

### DESCRIPTION OF EMBODIMENTS

### Anti-Netrin-4 antibody

The present invention provides an anti-Netrin-4 antibody that recognizes, as an epitope, an amino acid sequence of residues 192 to 202 in an amino acid sequence of human Netrin-4 (SEQ ID NO: 1, Accession No.: NP_067052.2) (referred to as the "antibody of the invention" below). The amino acid residues 192 to 202 in the amino acid sequence of human Netrin-4 are TGGEVIFKALS (SEQ ID NO: 2). An antibody that binds to a peptide of the amino acid sequence TGGEVIFKALS (referred to as the "epitope sequence" below) may serve as the antibody of the invention. The antibody of the invention has been demonstrated to alleviate the symptoms of hyperesthesia when administered to neuropathic pain model rats (see Examples).

The antibody of the invention may be a polyclonal antibody or a monoclonal antibody. The polyclonal antibody can be produced and obtained, for example, in the following manner. A peptide containing the epitope sequence (SEQ ID NO: 2) is dissolved in PBS, and, if needed, further mixed with an appropriate amount of a conventional adjuvant (for example, Freund's complete adjuvant) to prepare an immunogen. A mammal (e.g., a mouse, a rat, a rabbit, a goat, a horse, etc.) is immunized with the immunogen. The immunization method is not limited to a particular one, but preferred is subcutaneous or intraperitoneal injection given once or repeatedly several times at appropriate intervals, for example. After the immunization, the blood is collected from the immunized animal, the serum is separated, and a polyclonal antibody fraction is purified, according to conventional methods, to produce the polyclonal antibody.

The monoclonal antibody can be obtained by fusing immune cells (e.g., splenocytes) from the immunized mammal with myeloma cells to produce a hybridoma, culturing the hybridoma and collecting an antibody from the culture. Alternatively, an antibody gene from the hybridoma can be cloned using a known genetic engineering technique, then the gene is inserted into a suitable vector, and the vector is transfected into host cells to produce a recombinant monoclonal antibody. The phage display technique can also be used for production of the monoclonal antibody.

The antibody of the invention in the form of a monoclonal antibody may be an antibody produced by a hybridoma cell line, Human Netrin-4(192-202)-6A1 (Accession No. NITE BP-03603). The hybridoma cell line, Human Netrin-4 (192-202)-6A1 has been internationally deposited in NITE Patent Microorganisms Depositary, National Institute of Technology and Evaluation under Accession No. NITE BP-03603 (Deposit Date: February 14, 2022).

The antibody produced by the hybridoma cell line, Human Netrin-4(192-202)-6A1, has a heavy chain of the amino acid sequence set forth in SEQ ID NO: 3 and a light chain of the amino acid sequence set forth in SEQ ID NO: 4. A heavy chain variable region of the antibody consists of the amino acid sequence set forth in SEQ ID NO: 5, and a light chain variable region of the antibody consists of the amino acid sequence set forth in SEQ ID NO: 6. The heavy and light chain variable regions further contain the following complementarity determining regions (CDRs):
a heavy chain CDR1 of the amino acid sequence (DYEMH) set forth in SEQ ID NO: 7,
a heavy chain CDR2 of the amino acid sequence (AIDPETGDTVYNQKFKG) set forth in SEQ ID NO: 8,
a heavy chain CDR3 of the amino acid sequence (GFSDHY) set forth in SEQ ID NO: 9,
a light chain CDR1 of the amino acid sequence (RSSQSLVHSSGNTFLH) set forth in SEQ ID NO: 10,
a light chain CDR2 of the amino acid sequence (KVSSRF S) set forth in SEQ ID NO: 11, and
a light chain CDR3 of the amino acid sequence (SQSTHIPYT) set forth in SEQ ID NO: 12.

Preferably, the antibody of the invention contains (a) a heavy chain variable region comprising a heavy chain CDR1 of the amino acid sequence of SEQ ID NO: 7, a heavy chain CDR2 of the amino acid sequence of SEQ ID NO: 8, and a heavy chain CDR3 of the amino acid sequence of SEQ ID NO: 9; and (b) a light chain variable region comprising a light chain CDR1 of the amino acid sequence of SEQ ID NO: 10, a light chain CDR2 of the amino acid sequence of SEQ ID NO: 11, and a light chain CDR3 of the amino acid sequence of SEQ ID NO: 12.

The antibody of the invention may be a human chimeric antibody or a humanized antibody. The term "human chimeric antibody" refers to an antibody consisting of heavy and light chain variable regions from a non-human animal antibody, and heavy and light chain constant regions from a human antibody. The term "humanized antibody" refers to an antibody prepared by grafting CDRs from a non-human animal antibody into the CDRs of a human antibody, and is also called a CDR-grafted antibody, a reshaped antibody, etc. The framework regions (FRs) of the humanized antibody are selected so that the CDRs can form a favorable antigen-binding site. If needed, the amino acid sequences of FRs in the variable regions of the humanized antibody may be substituted so that the CDRs can form a suitable antigen-binding site.

The constant regions of the chimeric antibody or the humanized antibody may be the constant regions from a human antibody. For example, Cγ1, Cy2, Cy3, and Cy4 can be used for the constant region of the heavy chain, and Cκ and Cλ can be used for the constant region of the light chain. The constant regions from a human antibody may be modified to improve the stability of the antibody or the stable production of the antibody. The human antibody used for the humanization of the antibody may be of any isotype, including, for example, IgG, IgM, IgA, IgE, or IgD, and is preferably IgG, more preferably IgG1 or IgG3, and particularly preferably IgG1. The amino acid sequences of the constant regions of a human antibody can be obtained from a known database (Protein Data Bank etc.).

The human chimeric antibody of the invention is preferably an antibody composed of a heavy chain variable region of the amino acid sequence set forth in SEQ ID NO: 5, a light chain variable region of the amino acid sequence set forth in SEQ ID NO: 6, and heavy and light chain constant regions from a human antibody. The heavy chain variable region may consists of the amino acid sequence of SEQ ID NO: 5 having deletion, substitution or addition of one or several amino acids in regions other than CDR1, CDR2 or CDR3. The light chain variable region may consists of the amino acid sequence of SEQ ID NO: 6 having deletion, substitution or addition of one or several amino acids in regions other than CDR1, CDR2 or CDR3. The term "deletion, substitution or addition of one or several amino acids" refers to deletion, substitution or addition of an appropriate number of amino acids that can be deleted, substituted or added (preferably 10 amino acids or less, more preferably 7 amino acids or less, or further preferably 5 amino acids or less) using a known mutant peptide preparation technique, such as site-specific mutation.

The humanized antibody of the invention is preferably an antibody prepared by grafting a heavy chain CDR1 of the amino acid sequence set forth in SEQ ID NO: 7, a heavy chain CDR2 of the amino acid sequence set forth in SEQ ID NO: 8, a heavy chain CDR3 of the amino acid sequence set forth in SEQ ID NO: 9, a light chain CDR1 of the amino acid sequence set forth in SEQ ID NO: 10, a light chain CDR2 of the amino acid sequence set forth in SEQ ID NO: 11, and a light chain CDR3 of the amino acid sequence set forth in SEQ ID NO: 12 into the CDRs of a human antibody.

The human chimeric antibody and the humanized antibody can be produced as a recombinant antibody using a known genetic engineering technique. For example, a gene encoding a heavy chain of the human chimeric antibody can be prepared by ligating a portion encoding a heavy chain variable region of an antibody gene cloned from a hybridoma to a gene encoding a heavy chain constant region of a human antibody. Similarly, a gene encoding a light chain of the human chimeric antibody can be prepared by ligating a portion encoding a light chain variable region of an antibody gene cloned from a hybridoma to a gene encoding a light chain constant region of a human antibody. For example, a gene encoding a heavy chain of the humanized antibody can be prepared by substituting a portion encoding the CDRs 1, 2 and 3 of a heavy chain variable region of an antibody gene cloned from a hybridoma with the corresponding CDR portions of a gene encoding a heavy chain of a human antibody. Similarly, a gene encoding a light chain of the humanized antibody can be prepared by substituting a portion encoding the CDRs 1, 2 and 3 of a light chain variable region of an antibody gene cloned from a hybridoma with the corresponding CDR portions of a gene encoding a light chain of a human antibody.

The present invention also include a functional fragment of the antibody of the invention. The "functional fragment" herein may be any portion (partial fragment) of the antibody of the invention and has the epitope sequence as described above, i.e., any portion that retains the function of recognizing the amino acid residues 192 to 202 (TGGEVIFKALS) in the amino acid sequence of human Netrin-4. In particular, the functional fragment of the antibody of the invention may be, for example, Fab, F(ab')₂, Fab', Fv, scFv, dsFv, a diabody, a nanobody, etc. Such functional fragments can be prepared by a known method.

### Polynucleotide

The present invention also provides a polynucleotide encoding a heavy chain variable region of the antibody of the invention, and a polynucleotide encoding a light chain variable region of the antibody of the invention. The polynucleotide of the invention is useful for production of a recombinant version of the antibody of the invention.

The term "polynucleotide" as used herein can be interchangeably used with the terms "gene", "nucleic acid" and "nucleic acid molecule." The polynucleotides of the invention can present in the form of a RNA (for example, an mRNA) or the form of a DNA (for example, a cDNA or genomic DNA). The DNA may be double-stranded or single-stranded. A single-stranded DNA or RNA may be a coding strand (sense strand) or a non-coding strand (antisense strand).

The polynucleotide encoding the heavy chain variable region of the invention may be, for example, a polynucleotide encoding a heavy chain variable region of the amino acid sequence set forth in SEQ ID NO: 5, or a polynucleotide of the nucleotide sequence set forth in SEQ ID NO: 6. The polynucleotide encoding the light chain variable region of the invention may be a polynucleotide encoding a light chain variable region of the amino acid sequence set forth in SEQ ID NO: 13, or a polynucleotide of the nucleotide sequence set forth in SEQ ID NO: 14.

A method for obtaining the polynucleotide of the invention may include a method using an amplifying technique, such as PCR. For example, primers are designed based on the sequences at the 5' and 3' ends of the nucleotide sequence set forth in SEQ ID NO: 13 or 14 (or their complementary sequences). PCR or a similar technique is performed using the primers and using cDNA prepared from RNA of Human Netrin-4(192-202)-6A1 hybridomas etc. as a template to amplify the DNA region flanked by the primers. In this manner, a large amount of DNA fragments containing the polynucleotide encoding the polypeptide of the invention are obtained.

### Expression vector

The present invention also provides an expression vector comprising the polynucleotide of the invention. The expression vector of the invention can be used to produce the antibody of the invention or a functional fragment thereof. The expression vector of the invention may be any expression vector that contains the polynucleotide of the invention as described above, but is preferably a plasmid vector carrying a sequence recognized by an RNA polymerase. When the expression vector is used to produce the antibody of the invention, the expression vector preferably further contains a polynucleotide encoding a heavy chain constant region and a polynucleotide encoding a light chain constant region, in addition to the polynucleotide encoding the heavy chain variable region and the polynucleotide encoding the light chain variable region. In particular, the expression vector preferably contains a polynucleotide encoding a heavy chain containing a polynucleotide encoding a heavy chain variable region ligated to a polynucleotide encoding a heavy chain constant region; and a polynucleotide encoding a light chain containing a polynucleotide encoding a light chain variable region ligated to a polynucleotide encoding a light chain constant region.

A preparation method of such a recombinant expression vector may include, but not limited to, methods using a plasmid, a phage, a cosmid, etc. The type of the expression vector is not limited to a particular one, and may be selected as appropriate from a vector that can be expressed in host cells. In particular, a promoter sequence that ensures the expression of the polynucleotide of the invention is selected as appropriate for the type of host cells used. The selected promoter sequence and the polynucleotide of the invention are then inserted into a plasmid etc. selected from various types to produce a vector, which may be used as an expression vector. Preferably, the expression vector contains at least one selectable marker. The selectable marker may be, for example, dihydrofolate reductase or a neomycin resistance gene when the host is eukaryote cells; or a tetracycline resistance gene or an ampicillin resistance gene when the host is *Escherichia coli* or other bacteria. Using such a selectable marker, successful introduction of the polynucleotide of the invention into host cells can be confirmed, and successful expression of the polynucleotide of the invention in host cells can be confirmed.

### Transformed cells

The present invention also provides a transformed cell comprising the expression vector of the invention. The transformed cell of the invention can be prepared from cells of various types of microorganisms, plants or animals, which are known to be used as host cells. Specific examples of the host cells include bacterial cells, such as *Escherichia coli;* yeast cells, such as budding yeast *Saccharomyces cerevisiae,* or fission yeast *Schizosaccharomyces pombe; Xenopus* oocytes; insect cells; and animal cells, such as CHO cells or COS cells.

A method for introducing the expression vector of the invention into host cells is not limited to a particular one, and a conventionally known method, including electroporation, the calcium phosphate method, the liposome method, or the DEAE-dextran method, can be suitably used. The transformed cell of the invention preferably stably expresses the protein encoded by the polynucleotide of the invention, or may transiently expresses the protein encoded by the polynucleotide of the invention.

### Pharmaceutical composition

The present invention also provides a pharmaceutical composition for preventing or treating pain, comprising the antibody or a functional fragment thereof as an active ingredient. The pharmaceutical composition of the invention can be administered to humans or non-human animals via an oral or parenteral route. The pharmaceutical composition of the invention can be formulated into an appropriate dosage form depending on the route of administration. **In** particular, the pharmaceutical composition of the invention can be formulated into various types of formulations, including, for example, granules, tablets, pills, capsules, syrups, emulsions, suspensions, injectables, infusions, topical agents, or suppositories. Such various types of formulations can be produced using, for example, commonly used excipients, fillers, binders, wetting agents, disintegrants, surface active agents, lubricants, dispersants, buffering agents, preservatives, solubilizing agents, antiseptics, colorants, flavoring agents, stabilizers according to a conventional method.

Examples of the excipients include lactose, fructose, glucose, corn starch, sorbitol, crystalline cellulose, sterilized water, ethanol, glycerol, physiological saline, buffer solution, etc. Examples of the disintegrants include starch, sodium alginate, gelatin, calcium carbonate, calcium citrate, dextrin, magnesium carbonate, synthetic magnesium silicate, etc. Examples of the binders include methyl cellulose, ethyl cellulose, gum arabic, gelatin, hydroxypropyl cellulose, polyvinylpyrrolidone, etc. Examples of the lubricants include talc, magnesium stearate, polyethylene glycol, hydrogenated vegetable oil, etc. Examples of the stabilizers include amino acids, such as arginine, histidine, lysine, and methionine; human serum albumin, gelatin, dextran 40, methyl cellulose, sodium sulfite, sodium metasulfite, etc. Examples of other additives include sirup, petrolatum, glycerol, ethanol, propylene glycol, citric acid, sodium chloride, sodium nitrite, sodium phosphate, etc.

A preferred route of administration is a parenteral route of administration, and preferred dosage forms include injectables, transnasal agents, transpulmonary agents, transdermal agents, etc. Injectables can be systemically or locally administered by, for example, intravenous injection, intramuscular injection, intraperitoneal injection, subcutaneous injection, etc. The mode of administration can be selected as appropriate for the age and/or symptoms of the patient. The dosage of the pharmaceutical composition of the invention may be, for example, selected from the range of 0.0001 mg to 1000 mg of the active ingredient per kilogram of the body weight for a single dose, but the dosage is not limited thereto. The dosage and the mode of administration may vary depending on the body weight, age, symptoms, etc. of the patient, and can be selected as appropriate by the person skilled in the art.

### Vaccine composition for preventing or treating pain

The present invention also provides a vaccine composition for preventing or treating pain, comprising a peptide comprising an amino acid sequence of residues 192 to 202 (SEQ ID NO: 2) in an amino acid sequence of human Netrin-4 (SEQ ID NO: 1), and a carrier protein (referred to as the "vaccine composition of the invention" below). Administration of the vaccine composition of the invention induces, in vitro, the production of the anti-Netrin-4 antibody that recognizes, as an epitope, an amino acid sequence of residues 192 to 202 (SEQ ID NO: 2) in an amino acid sequence of human Netrin-4 (SEQ ID NO: 1), thereby preventing or treating pain.

The peptide containing an amino acid sequence of residues 192 to 202 in an amino acid sequence of human Netrin-4 (SEQ ID NO: 1) (referred to as the "antigenic peptide" below) contained in the vaccine composition of the invention may be a peptide of the amino acid sequence TGGEVIFKALS, or a fragment of human Netrin-4 containing the amino acid sequence TGGEVIFKALS. The length of the fragment is not limited to a particular one, and may be, for example, 20 amino acids or less, 18 amino acids or less, 16 amino acids or less, 15 amino acids or less, 14 amino acids or less, 13 amino acids or less, or 12 amino acids or less in length. The amino acid sequence of the antigenic peptide may be an amino acid sequence having an additional single amino acid (e.g., cysteine) at the N- or C-terminus to be linked (conjugated) to a carrier protein via a linker.

The carrier protein is not limited to a particular one, and can be selected as appropriate from known carrier proteins for use in a vaccine. Examples of known carrier proteins include, for example, albumin, ovalbumin, keyhole limpet hemocyanin (KLH), pseudomonas exotoxin, tetanus toxin, ricin toxin, diphtheria toxin, cholera toxin, heat-labile enterotoxin, epidermal growth factor, fibroblast growth factor, transferrin, platelet derived growth factor, poly-L-lysine, poly-L-glutamine, mannose-6-phosphate, hepatitis B virus core protein, etc.

The antigenic peptide is preferably linked (conjugated) to the carrier protein. The peptide and the carrier protein may be directly linked as in a fusion protein, or may be linked via a linker (defined to have the same meaning as a spacer). The linker may be any linker that can link the antigenic peptide to the carrier protein. For example, aminocarboxylic acids, such as β-aminoalanine, γ-aminobutyric acid, ε-aminocaproic acid, 7-aminoheptanoic acid, 12-aminolauric acid, glutamic acid, or p-aminobenzoic acid can be used. L-amino acids, which are present in naturally occurring proteins, or D-amino acids of such amino acids can be used. Crosslinkers, such as EMCS (N-(6-maleimidocaproyloxy)succinimide), glutaraldehyde, or Sulfo GMBS (N-γ-maleimidobutyryl-oxysulfosuccinimide ester) can also be used.

A complex (conjugate) of the antigenic peptide and the carrier protein is preferably acetylated at the N-terminal amino acid. The complex is also preferably amidated at the C-terminal amino acid. More preferably, the complex is acetylated at the N-terminal amino acid and amidated at the C-terminal amino acid.

The vaccine composition of the invention may further contain one or more adjuvants. The adjuvants can be selected as appropriate from known adjuvants. Specific examples of the adjuvants include aluminum adjuvants (e.g., aluminum salts, such as aluminum hydroxide, aluminum phosphate, or aluminium sulfate, or a combination thereof), Freund's adjuvant (complete or incomplete), TLR ligands (e.g., CpG, Poly(I:C), Pam3CSK4, etc.), BAY, DC-chol, pcpp, monophosphoryl lipid A, QS-21, cholera toxin, formyl methionyl peptide, etc.

The vaccine composition of the invention can be administered via an oral or parenteral route. The parenteral route of administration may include, for example, intraperitoneal, subcutaneous, intracutaneous, intramuscular, intravenous, intranasal, transdermal, transmucosal, sublingual, and inhalation administrations. A preferred route of administration is a parenteral route of administration, and is more preferably intracutaneous, subcutaneous, or intramuscular administration.

The vaccine composition of the invention can be formulated by blending the antigenic peptide, a carrier protein, and a pharmaceutically acceptable carrier, and optionally an additive as appropriate. In particular, the vaccine composition can be formulated into an oral formulation, such as a tablet, a coated tablet, a pill, a powder, granules, a capsule, a solution, a suspension or an emulsion; or a parenteral formulation, such as an injectable, an infusion, a suppository, an ointment or a patch. The blending ratio of the carrier or additive is determined as appropriate based on the amount usually used in the pharmaceutical field. The carrier or additive that can be combined is not limited to a particular one, and examples thereof include various types of carriers, such as water, physiological saline, other aqueous solvents, or aqueous or oily bases; and various types of additives, such as excipients, binders, pH adjusting agents, disintegrants, absorption enhancers, lubricants, colorants, flavor improvers or fragrances.

The number of doses and dose intervals of the vaccine composition of the invention are not limited to a particular one. For example, the vaccine composition of the invention may be administered at a single dose, or may be administered at multiple doses at intervals of about 2 days to about 8 weeks. The dosage of the vaccine composition varies depending on the subject that receives the vaccine composition, the mode of administration, etc. Preferably, the dosage is selected such that about 0.01 µg to about 10 mg of the antigenic peptide is administered at a single dose, more preferably about 0.1 µg to about 1 mg of the antigenic peptide is administered at a single dose, or further preferably about 1 µg to about 0.1 mg of the antigenic peptide is administered at a single dose.

The present invention further includes the following.

A method for treating pain, comprising administering the antibody of the invention or a functional fragment thereof.

The antibody of the invention or a functional fragment thereof for use in prevention or treatment of pain.

Use of the antibody of the invention or a functional fragment thereof in the production of a pharmaceutical composition for preventing or treating pain.

### EXAMPLES

The present invention will be described in more detail below with reference to Examples, but the present invention is not limited thereto.

### Example 1: Effect of anti-Netrin-4 polyclonal antibody in neuropathic pain model rats

### (1) Preparation of polyclonal antibody

A synthetic peptide of amino acid residues 192 to 202 (TGGEVIFKALS, SEQ ID NO: 2) in the amino acid sequence of human Netrin-4 (SEQ ID NO: 1) was used as an antigen. Two SPF rabbits were immunized with the antigen by a conventional method to produce a polyclonal antibody. The antibody was purified using a peptide column.

### (2) Production of neuropathic pain model rats

Neuropathic pain model rats were produced by partial ligation of the sciatic nerve (see non patent literature 5). Under anesthesia with 2.0% isoflurane inhalation solution, a polyethylene catheter tube was inserted between the fourth and fifth vertebra for intrathecal administration of the antibody (Day -7). Seven days after catheter insertion (Day 0), rats were anesthetized with 2.0% isoflurane inhalation solution, and shaved on the left hind-limb thigh and around the groin. The skin and muscle over the joint portion of the thigh and hip bones were incised, and the sciatic nerve, which runs along with the thigh bone, was exposed. A half or one-third portion of the sciatic nerve was ligated with a 4-0 nylon suture, and the muscle and skin were then sutured. In the contralateral (right) hind limb, the skin and muscle over the joint portion of the thigh and hip bones were incised, and the sciatic nerve was exposed and the muscle and skin were then sutured, in the same manner as in the left hind limb, but the sciatic nerve was kept intact for use as a sham surgery.

### (3) Antibody administration

Seven days after partial sciatic nerve ligation surgery (Day 7), 30 µl of the anti-Netrin-4 polyclonal antibody (1 µg/µl) was intrathecally administered. To control group, 30 µl of mouse control IgG (1 µg/µl) was intrathecally administered.

### (4) Evaluation of pain-related behavior

The von Frey filament test was used to measure the response to mechanical stimulation. A plastic case was placed on a metallic mesh, and the neuropathic pain model rats were placed in the plastic case and habituated for 5 to 10 minutes until settled. A filament (Semmes-Weinstein Von Frey Anesthesiometer, Muromachi Kikai Co., Ltd.) was applied to the center of the plantar surface of the hind paw for 3 to 5 seconds, and the threshold for hind paw withdrawal (g) was measured. The withdrawal thresholds of both paws were measured from the day of catheter insertion (Day -7) to day 4 post-antibody administration (Day 11).

### (5) Results

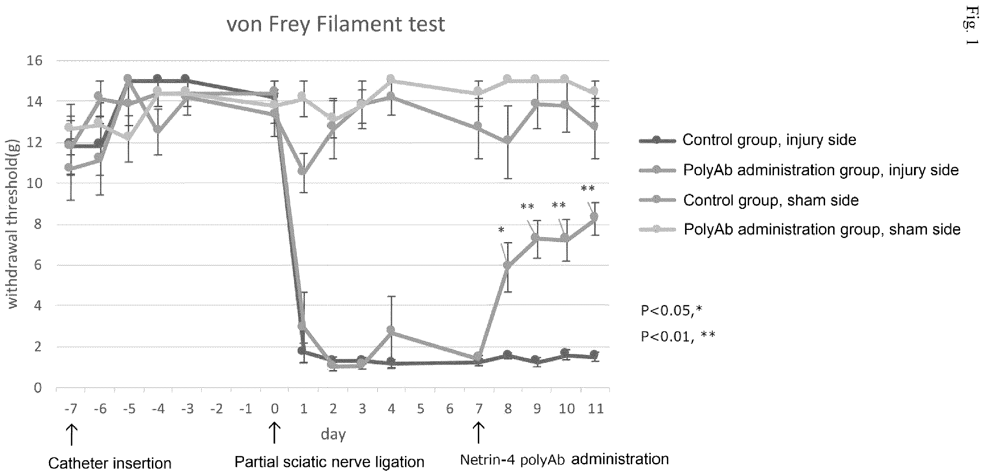

The results are shown in Fig. 1. Administration of the anti-Netrin-4 polyclonal antibody to the neuropathic pain model rats, which had decreased withdrawal thresholds after the partial sciatic nerve ligation surgery, significantly improved withdrawal thresholds as compared with control group that received control IgG. It was demonstrated that administration of the anti-Netrin-4 polyclonal antibody alleviates the symptoms of hyperesthesia due to the partial sciatic nerve ligation.

### Example 2: Effect of anti-Netrin-4 monoclonal antibody in neuropathic pain model rats

### 2-1 Preparation of anti-Netrin-4 monoclonal antibody

### (1) Epitope peptide

A peptide, C-Ahx-TGGEVIFKALS, containing amino acid residues 192 to 202 (TGGEVIFKALS, SEQ ID NO: 2) in the amino acid sequence of human Netrin-4 (SEQ ID NO: 1) was synthesized. This epitope peptide was linked to bovine thyroglobulin as a carrier protein.

### (2) Preparation of monoclonal antibody

Four mice were immunized with the epitope peptide. After four immunizations, the blood was collected to determine the antibody titers using ELISA against recombinant human Netrin-4 and TGGEVIFKALS peptide. Mouse lymphocytes with increased antibody titers were fused with myeloma cells, and cultured in HAT medium to produce hybridomas. About ten days after cell fusion, antibody production in the culture supernatant was screened by ELISA (against recombinant human Netrin-4 and TGGEVIFKALS peptide), and 95 positive wells were selected. Primary cloning was then performed by limiting dilution, followed by screening by ELISA (against recombinant human Netrin-4 and TGGEVIFKALS peptide). Secondary cloning was then performed by limiting dilution, followed by screening by ELISA (against recombinant human Netrin-4 and TGGEVIFKALS peptide). A single clone was selected. The selected clone was expanded, and cultured at a high density in a serum-free medium. The culture medium was collected and purified on a protein A column to produce IgG. Purity was measured using AKTA-FPLC, and the titer was measured by ELISA. IgG was further purified with low endotoxin levels and used for the subsequent experiments.

The selected hybridoma clone (Human Netrin-4 (192-202)-6A1) has been internationally deposited in NITE Patent Microorganisms Depositary, National Institute of Technology and Evaluation under Accession No. NITE BP-03603 (Deposit Date: February 14, 2022).

### 2-2 Effect of intrathecal administration of anti-Netrin-4 monoclonal antibody

Effect of the anti-Netrin-4 monoclonal antibody in the pain model rats was evaluated in the same manner as in Example 1 except that the anti-Netrin-4 monoclonal antibody (1 µg/µl) was used in place of the anti-Netrin-4 polyclonal antibody of Example 1.

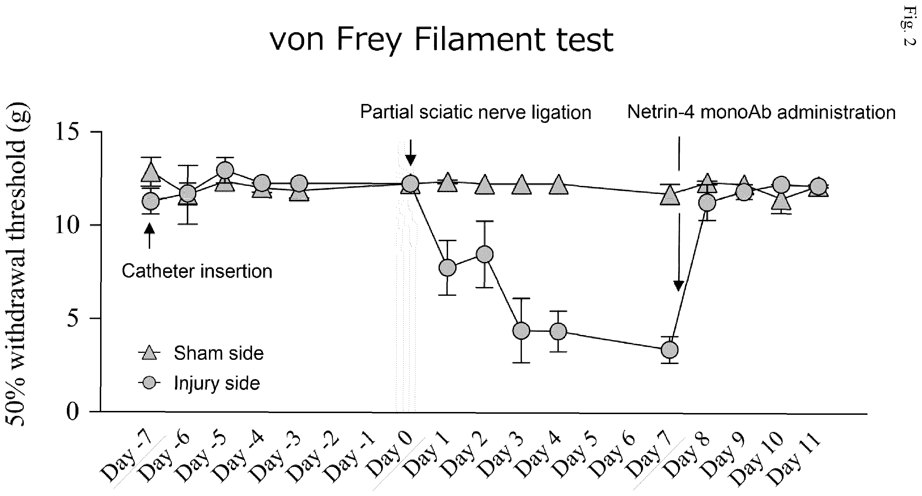

The results are shown in Fig. 2. Intrathecal administration of the anti-Netrin-4 monoclonal antibody to the neuropathic pain model rats, which had decreased withdrawal thresholds after the partial sciatic nerve ligation surgery, improved the withdrawal thresholds at the injury side to almost the same level as that at the sham side. It was demonstrated that the pain alleviating effect of the anti-Netrin-4 monoclonal antibody is significantly higher than that of the anti-Netrin-4 polyclonal antibody in Fig. 1.

### 2-3 Effect of administration of anti-Netrin-4 monoclonal antibody to dorsal root ganglion

The von Frey filament test was performed on Day 0, and partial ligation surgery was performed on the sciatic nerve of the left hind limb of rats in the same manner as in Example 1. The sciatic nerve of the right hind limb was exposed without ligation and served as the sham side. Seven days after partial sciatic nerve ligation surgery (Day 7), 3 µl of the anti-Netrin-4 monoclonal antibody (1 µg/µl) was administered to the dorsal root ganglion of the rats manually using a glass capillary tube. The von Frey filament test was performed from Day 0 to Day 11 to measure the withdrawal thresholds for both hind paws.

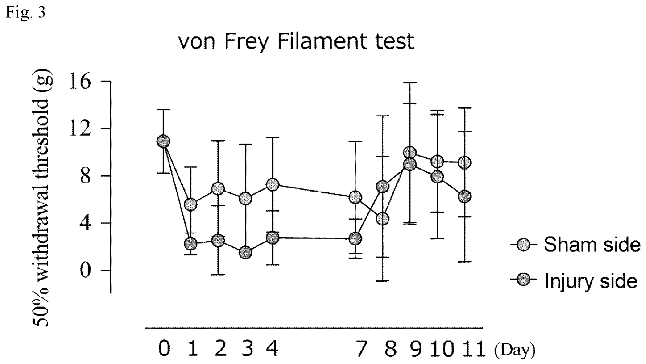

The results are shown in Fig. 3. Administration of the anti-Netrin-4 monoclonal antibody to the dorsal root ganglion of the neuropathic pain model rats, which had decreased withdrawal thresholds after the partial sciatic nerve ligation surgery, improved the withdrawal thresholds at the injury side to almost the same level as that at the sham side. The results revealed that administration of the antibody to the dorsal root ganglion also improves pain after partial sciatic nerve ligation.

The present invention is not limited to each of the embodiments and Examples as described above, and various modifications are possible within the scope of the claims. Embodiments obtainable by appropriately combining the technical means disclosed in the different embodiments of the present invention are also included in the technical scope of the present invention. The contents of the scientific literature and the patent literature cited herein are hereby incorporated by reference in their entirety.

### ACCESSION NUMBER

Identification of the microorganism
   Identification reference: Human Netrin-4(192-202)-6A1
   Accession number.: NITE BP-03603
Deposit Date
   February 14, 2022
Depositary authority
   Name: NITE Patent Microorganisms Depositary, National Institute of Technology and Evaluation
   Address: #122, 2-5-8 Kazusakamatari, Kisarazu-shi, Chiba 292-0818, Japan

## Claims

1. An anti-Netrin-4 antibody that recognizes, as an epitope, an amino acid sequence of residues 192 to 202 (SEQ ID NO: 2) in an amino acid sequence of human Netrin-4 (SEQ ID NO: 1).

2. The anti-Netrin-4 antibody according to claim 1, wherein the antibody comprises the following (a) and (b):
(a) a heavy chain variable region comprising a heavy chain CDR1 of the amino acid sequence of SEQ ID NO: 7, a heavy chain CDR2 of the amino acid sequence of SEQ ID NO: 8, and a heavy chain CDR3 of the amino acid sequence of SEQ ID NO: 9; and
(b) a light chain variable region comprising a light chain CDR1 of the amino acid sequence of SEQ ID NO: 10, a light chain CDR2 of the amino acid sequence of SEQ ID NO: 11, and a light chain CDR3 of the amino acid sequence of SEQ ID NO: 12.

3. The antibody according to claim 1 or 2, wherein the antibody is a human chimeric antibody or a humanized antibody.

4. The antibody according to claim 2, wherein the antibody is produced by a hybridoma cell line, Human Netrin-4(192-202)-6A1 (Accession No. NITE BP-03603).

5. A hybridoma cell line, Human Netrin-4(192-202)-6A1 (Accession No. NITE BP-03603).

6. A functional fragment of the antibody according to claims 1 to 4.

7. A polynucleotide encoding the heavy chain variable region of the antibody according to claims 1 to 4.

8. A polynucleotide encoding the light chain variable region of the antibody according to claims 1 to 4.

9. An expression vector comprising the polynucleotide according to claim 7 and/or claim 8.

10. A transformed cell comprising the expression vector according to claim 9.

11. A pharmaceutical composition for preventing or treating pain, comprising the antibody according to claims 1 to 4 or the functional fragment according to claim 6 as an active ingredient.

12. The pharmaceutical composition according to claim 11, wherein the composition is for administration to dorsal root ganglion.

13. A vaccine composition for preventing or treating pain, comprising a peptide comprising an amino acid sequence of residues 192 to 202 (SEQ ID NO: 2) in an amino acid sequence of human Netrin-4 (SEQ ID NO: 1), and a carrier protein.
